# FASCICULE DE BREVET EUROPEEN

(11) **EP 3 466 470 B1**
(45) Date de publication et mention de la délivrance du brevet: **04.08.2021**
(21) Numéro de dépôt: 17195526.3
(22) Date de dépôt: 09.10.2017
(51) Int. Cl.: A61M 16/04

(54) **RÉGULATEUR DE PRESSION POUR BALLONNET DE SONDE D'INTUBATION, ET SYSTÈME DE RESPIRATION COMPORTANT UN TEL RÉGULATEUR**
DRUCKREGULATOR FÜR CUFF AN TRACHEALTUBEN, UND ATMUNGSSYSTEM MIT EINEM SOLCHEN REGULATOR
PRESSURE REGULATOR FOR BALLOON OF AN INTUBATION CATHETER, AND BREATHING SYSTEM COMPRISING SUCH A REGULATOR

(43) Date de publication de la demande: 10.04.2019
(73) Titulaire: Leved, 75011 Paris (FR)
(72) Inventeur: LEVÊQUE, Edouard, 75011 PARIS (FR)
(74) Mandataire: Gauchet, Fabien Roland

(56) Documents cités:
- EP-A1- 0 489 516
- WO-A1-99/33508
- WO-A1-99/40960
- WO-A1-02/074376
- WO-A1-2013/102905
- WO-A1-2013/139986
- FR-A1- 2 940 621
- US-A1- 2011 109 458

## Description

Régulateur de pression pour ballonnet de sonde d'intubation, système de respiration comportant un tel régulateur et récipient comportant une chambre à volume variable pour un tel régulateur.

La présente invention concerne un régulateur de pression pour ballonnet de sonde d'intubation, canule de trachéotomie ou instrument similaire destiné à la ventilation d'un patient, ledit instrument comportant un tuyau principal ayant une première extrémité destinée à être insérée dans la trachée artère d'un patient et une seconde extrémité destinée à être raccordée à un appareil respirateur, un ballonnet annulaire gonflable d'étanchéité, qui entoure la première extrémité du tuyau principal, et au moins un tuyau auxiliaire ayant une première extrémité raccordée au ballonnet d'étanchéité et une seconde extrémité munie d'une valve pouvant être raccordée à une source d'air comprimé pour le gonflage du ballonnet d'étanchéité, ledit régulateur de pression comprenant un récipient définissant une chambre à volume variable ayant au moins un premier orifice pour être raccordé audit tuyau auxiliaire de telle façon que, en service, la chambre à volume variable du régulateur de pression et le ballonnet d'étanchéité soient en communication, ainsi qu'un moyen de pression agissant sur le récipient pour exercer une certaine force au moyen d'une masse pouvant se déplacer le long d'une tringle articulée pour régler la pression de l'air contenu dans ladite chambre à volume variable, et par suite la pression de l'air contenu dans le ballonnet d'étanchéité.

L'invention concerne aussi également un système de respiration comportant un tel régulateur.

Tout patient qui subit une anesthésie générale, qui est dans un état comateux ou souffre d'une insuffisance respiratoire doit être impérativement ventilé. Pour cela, on utilise habituellement une machine appelée « respirateur » ou « machine à ventiler », qui a pour fonction d'envoyer sous pression un mélange d'oxygène et de gaz carbonique, éventuellement mélangé avec un gaz anesthésiant, dans les poumons du patient. Ce mélange gazeux et la machine vont suppléer aux mouvements musculaires qui permettent au patient d'inspirer et d'expirer.

Le mélange gazeux peut être insufflé dans la trachée-artère du patient au moyen d'un tuyau (tuyau principal) qui passe soit par la bouche, soit par le nez du patient et qui est usuellement appelé « sonde d'intubation endo-trachéale » ou « sonde d'intubation endo-nasale » selon le cas. Si le patient doit être ventilé sur une longue durée (plus de quinze jours) ou si l'intubation est rendue impossible par des problèmes d'anatomie ou de traumatisme de la face, les chirurgiens ont habituellement recours à une « canule de trachéotomie ». Cette canule, plus ou moins rigide, de forme coudée, est constituée, comme les sondes d'intubations précitées, par un tuyau (tuyau principal) qui est introduit dans la trachée-artère du patient en pratiquant une ouverture dans la trachée (trachéotomie).

Lorsque le patient est ventilé par une sonde d'intubation ou une canule de trachéotomie, il est impératif de fermer hermétiquement l'accès par lequel est envoyé le mélange gazeux afin que celui puisse gonfler la cage thoracique du patient et rester dans ses poumons assez longtemps pour que l'échange oxygène/gaz carbonique s'effectue. Pour cela, le tuyau principal de la canule de trachéotomie ou de la sonde d'intubation est usuellement pourvu, dans la région de son extrémité qui est introduite dans la trachée-artère, d'un petit ballon annulaire gonflable, appelé « ballonnet » qui entoure l'extrémité précitée du tuyau principal. Une fois que la canule ou la sonde a été mise en place, ce ballonnet est gonflé à l'air à l'aide d'un petit tuyau (tuyau auxiliaire) qui est en partie incrusté dans la matière du tuyau principal et qui permet le passage de l'air entre le ballonnet et l'extérieur. L'extrémité extérieure du tuyau auxiliaire est habituellement pourvue d'une valve (clapet anti-retour), à laquelle peut être raccordée une source d'air comprimé, comme par exemple, une poire en caoutchouc actionnable à la main ou une seringue pour le gonflage du ballonnet. Cette valve empêche l'air de ressortir du ballonnet après que celui-ci a été gonflé et que la poire de gonflage en caoutchouc ou la seringue a été enlevée. Pour remplir sa fonction d'étanchéité, le ballonnet doit avoir une pression interne adaptée afin qu'il soit hermétiquement plaqué contre la trachée-artère du patient afin de retenir le mélange insufflé dans les poumons tout le temps de l'insufflation. De plus, si le ballonnet n'est pas assez gonflé, des sécrétions peuvent descendre vers les poumons et entraîner ainsi des risques d'infections. Il est donc indispensable que le ballonnet soit correctement appliqué contre la paroi trachéale.

Toutefois, la pression qu'exerce le ballonnet de la sonde d'intubation ou de la canule de la trachéotomie sur la paroi trachéale n'est pas sans conséquence. En effet, toute pression continue sur une partie quelconque du corps humain gêne la circulation du sang dans la partie concernée. Si la pression exercée par le ballonnet sur la paroi trachéale est trop importante, cette pression empêchera la circulation sanguine dans la zone de la paroi trachéale qui est en contact avec le ballonnet. Cette absence de circulation sanguine, si elle se prolonge, peut entraîner une nécrose de la zone précitée de la trachée. Cette nécrose peut elle-même provoquer une sténose de la trachée, c'est-à-dire que son diamètre se réduit et que la trachée se resserre autour du ballonnet. Bien que l'air soit un élément compressible, en cas de sténose et pour chaque fluctuation du diamètre de la trachée (lors de chaque insufflation et exsufflation), il se produit une surpression dans le ballonnet qui gêne l'irrigation sanguine des tissus dans cette zone de la trachée. En effet, le volume d'air contenu dans le ballonnet oppose une résistance au resserrement de la trachée et cette résistance se traduit par une pression accrue du ballonnet contre la paroi trachéale, ce qui amplifie le phénomène de non-vascularisation, donc les risques de nécrose et de sténose.

D'autre part, lors d'une anesthésie générale, le gaz utilisé pour l'anesthésie, usuellement du protoxyde d'azote, possède une composition moléculaire telle que le gaz insufflé par le tuyau principal dans les poumons peut migrer dans le ballonnet d'étanchéité à travers la paroi de celui-ci. En rentrant dans le ballonnet, le gaz augmente le volume de ce dernier et cet accroissement de volume provoque une augmentation de la pression exercée par le ballonnet sur la trachée. Là encore, il y a donc un risque d'absence de vascularisation de la zone de la paroi trachéale qui est en contact avec le ballonnet, d'où un risque de nécrose et de sténose de la trachée.

Quand les ballonnets sont correctement gonflés, les sécrétions oro-pharyngées, non aspirées par le personnel médical, sont retenues au-dessus du ballonnet qui les empêche de descendre dans les poumons.

Cependant, pendant les périodes où le ballonnet est insuffisamment gonflé, les sécrétions au-dessus du ballonnet s'écoulent dans les poumons. Ces écoulements répétés provoquent des infections pulmonaires chez ces patients, infections pulmonaires, qui sont la première cause de décès en réanimation.

De manière à résoudre ces problèmes, on a proposé dans le document de brevet WO 99/40960, un régulateur de pression pour sonde d'intubation, canule de trachéotomie ou instrument similaire qui permet de maintenir une pression constante et réglable de l'air contenu dans le ballonnet d'étanchéité de l'instrument et sensiblement constante mais réglable sur une paroi dudit récipient pour régler la pression de l'air contenu dans ladite chambre à volume variable et par suite, la pression de l'air contenu dans le ballonnet d'étanchéité.

Un tel régulateur de pression s'est révélé particulièrement efficace pour maintenir le volume du ballonnet de manière suffisante pour le maintenir en appui sur la paroi trachéale sans prendre le risque d'engendrer une blessure de ladite trachée. En effet, il permet de maintenir une pression constante dans le ballonnet, cette pression étant facilement réglable à l'aide d'une masse déplaçable le long d'une tringle en appui sur le récipient rempli d'air et relié au ballonnet. (CF Etude clinique Docteur Duguet, réanimation pulmonaire, Pitié-Salpétriere, Paris France et publiée en novembre 2007 dans la revue « Intensive care medecin »).

On a proposé aussi dans le document de brevet FR2940621 un régulateur de pression impliquant la présence d'un ressort qui ne présente pas une fiabilité suffisante. Aussi, la présente invention a pour but de proposer un régulateur de pression du type permettant d'assurer et de maintenir constante une pression préétablie dans le ballonnet de sorte à se prémunir notamment de manipulations intempestives qui pourraient survenir dans l'entourage du patient.

Pour cela, un tel régulateur est remarquable en ce qu'il est prévu un système de verrouillage de ladite masse pour éviter son déplacement inopiné, système de verrouillage constitué par un élément vissable muni d'une tête de friction pour être mis en contact de blocage sur une partie de ladite tringle par un mouvement de rotation et d'une partie filetée pénétrant dans le corps de la masse.

La description suivante accompagnée des dessins ci-annexés, le tout donné à titre d'exemple non limitatif fera bien comprendre comment l'invention peut être réalisée, sur les dessins :
La figure 1 représente un régulateur conforme à l'invention faisant partie d'un système respirateur,
la figure 2 représente une coupe du régulateur conforme à l'invention,
la figure 3 représente une vue en perspective avant du régulateur de pression selon la figure 1,
la figure 4 montre une coupe de la masse avec la tringle,
la figure 5 montre une coupe transversale de la tringle,
la figure 6 montre, d'une manière détachée, les éléments constitutifs d'une variante de réalisation du récipient,
la figure 7 montre le récipient de la figure 6 après son montage.

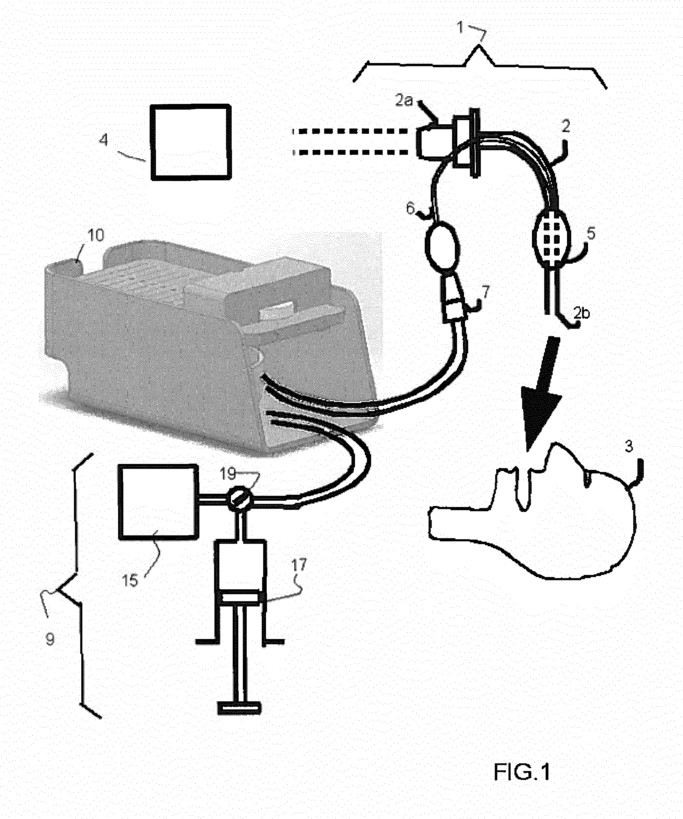

Comme on peut le voir à la figure 1, la canule de trachéotomie 1 comporte un tuyau principal 2 de forme arquée en une matière élastomère flexible appropriée à une utilisation médicale ou chirurgicale, dont une première extrémité 2b est destinée à être insérée dans la trachée artère d'un patient 3 et dont la seconde extrémité 2a est destinée à être raccordée à un appareil respirateur représenté schématiquement par le bloc 4. Dans la région de son extrémité 2a, un ballonnet annulaire d'étanchéité 5 entoure ladite première extrémité 2a du tuyau principal 2, ledit ballonnet étant gonflable par l'intermédiaire d'un tuyau auxiliaire souple 6 ayant une première extrémité raccordée au ballonnet d'étanchéité 5 et une seconde extrémité munie d'une valve anti-retour 7 raccordée à une source d'air comprimé 9 comme par exemple une pompe actionnable à la main, telle qu'une poire en caoutchouc, pour le gonflage du ballonnet 5. Cette canule 1 est un instrument bien connu, disponible dans le commerce et qui ne sera pas décrit plus en détails.

Le régulateur de pression 10 est intercalé entre la source d'air comprimé 9 et le tuyau auxiliaire 6. La source d'air comprimé peut être formé d'un générateur d'air comprimé 15 et d'une seringue 17. Le tuyau auxiliaire 6 est alimenté à partir de ces éléments 15 et 17 en fonction de la commande d'un robinet 19. La seringue 17 fournit l'air lors de la mise en route du régulateur. Les différentes connexions sont effectuées en utilisant des tuyaux de couleurs différentes.
Selon un aspect de l'invention, le régulateur peut être incorporé dans un bâti avec le l'appareil respirateur 4 de sorte à former un système respirateur compact,

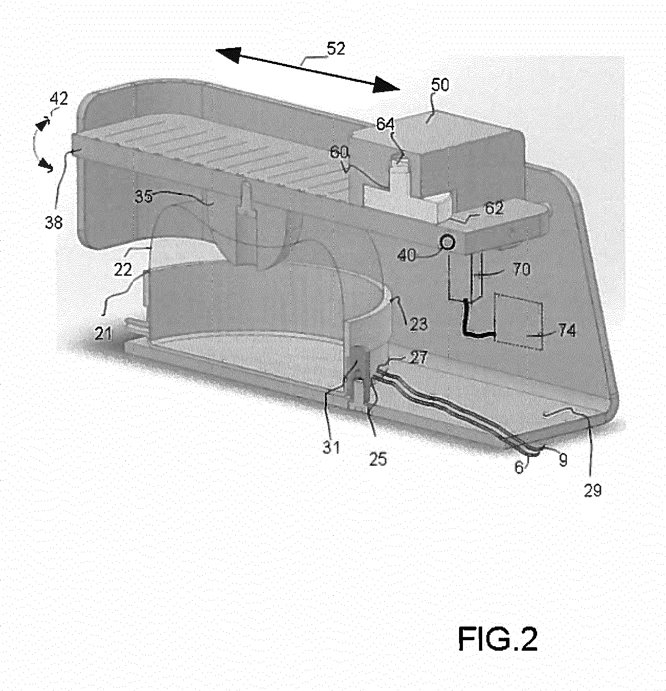

La figure 2 représente en coupe le régulateur conforme à l'invention. Il comprend un récipient 21 formé d'une chambre à volume variable 22 et d'une embase 23. Ce récipient 21 est muni d'un au moins un premier orifice 25 raccordé ou raccordable audit tuyau auxiliaire 6 de la canule 1, de telle façon que, en service, la chambre à volume variable du régulateur de pression et le ballonnet d'étanchéité 5 soient en communication. Un second orifice 27 est raccordé à la source 9, Le récipient 21 est maintenu sur la base 29 du régulateur dans une bonne position au moyen de trois picots dont un seul portant la référence 31 est montré sur cette figure 2. De préférence les connexions de ces tuyaux d'air s'effectuer à l'aide d'embouts LUER.

De préférence, pour des raisons d'hygiène, le récipient 21 est réalisé sous la forme d'un élément jetable, à patient unique, tel qu'une poche en un matériau souple et imperméable et de forme similaire à une poche de perfusion.

De préférence, les tuyaux reliés au ballonnet seront collés et non entrés en force. On évite ainsi des coupures d'air qui peuvent être très préjudiciables au patient. En outre, pour éviter de mauvais branchements des tuyaux auxiliaires les différentes couleurs sont affectées aux fonctions des différents tuyaux de sorte que, le branchement étant effectué correctement, on constate une continuité dans les couleurs.

Pour définir avec précision et constance la pression régnant à l'intérieur de la chambre 22 on utilise un piston 35 faisant pression sur le haut de la chambre 22. Ce piston 35 est sous la dépendance d'une tringle 38 pouvant pivoter selon un axe 40 comme cela est indiqué par la double flèche 42. Sur cette tringle une masse 50 peut se déplacer selon la double flèche 52. La fixation de cette masse sur la tringle sera explicitée plus loin dans la présente description.

Selon l'invention, le déplacement de cette masse 50 peut être bloqué ou verrouillé de sorte qu'un déplacement inopiné peut être évité. Ce blocage est obtenu au moyen d'une vis 60 dont la tête 62 frotte sur la surface de la tringle et la partie filetée 64 pénètre dans le corps de la masse 50. En manœuvrant la tête de vis 62, compte tenu du fait que la masse 50 est maintenue plaquée contre la tringle 38, on obtient ainsi le blocage de cette masse à une position donnée le long de la tringle. La position de cette masse fixe la pression de l'air contenu dans le récipient 21 et de là, la pression au sein du ballonnet 5 .

Selon un autre aspect de l'invention, on a associé à la tringle 38 un détecteur de déplacement 70 couplé à un système électronique qui fournit différentes informations à, notamment, une unité d'affichage 74 telles que la pression et l'historique des manipulations qui ont été effectuées au moyen de l'appareil respirateur.

Les documents de brevet WO2013/102905 et US2011/109458 décrivent aussi des systèmes de surveillance mais n'apportent aucun enseignement sur la façon de réguler la pression.

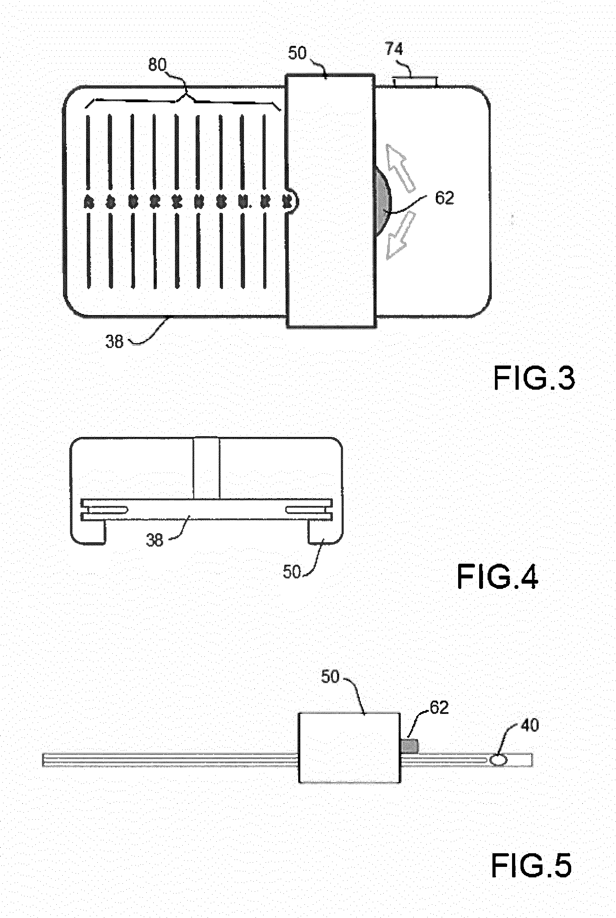

La figure 3 montre, vue du haut, la tringle 38 sur laquelle est disposée la masse 50. La figure 3 montre qu'un index de pression 80 est gravé sur la face de la tringle 38. La tête de la vis de blocage est accessible de sorte qu'il est possible pour un opérateur de bloquer et de débloquer le déplacement de la masse 50.

La figure 4 montre bien que la masse 50 présente une forme en étrier qui enserre la tringle 38. De cette façon la masse est solidaire de cette tringle 38 mais son déplacement longitudinal est rendu possible en position de déblocage.

La figure 5 montre une vue transversale de la tringle 38.

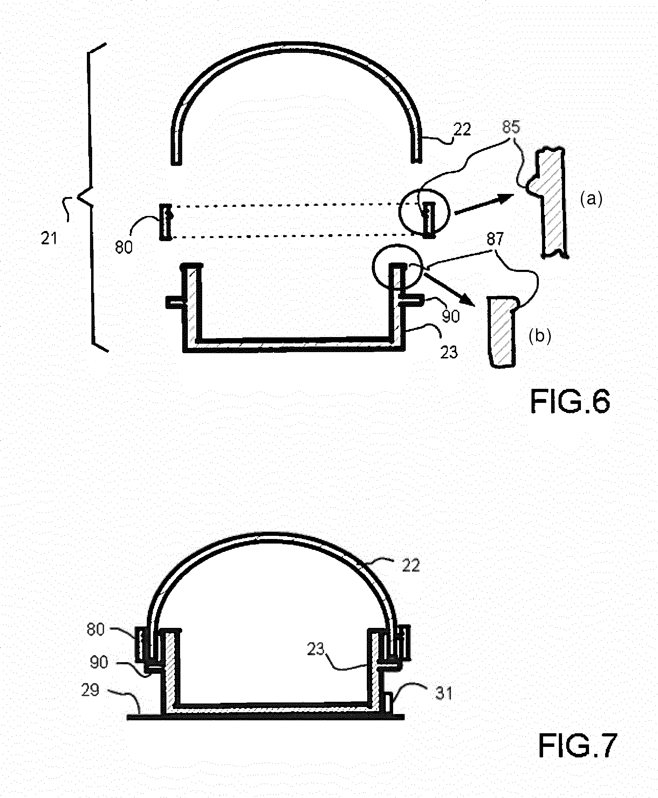

Selon un aspect de l'invention, on propose comme cela est montré aux figures 6 et 7 un mode de réalisation préféré d'un mode de réalisation du récipient 21. Ce récipient est formé de trois éléments dont deux ont déjà été cités : la chambre à volume variable 22 et l'embase 23. La chambre à volume variable 22 est réalisée en une matière plastique transparente et souple et vient se glisser sur le bord supérieur de l'embase 23. Pour assurer une fixation robuste on a prévu une bague 80 qui enserre la chambre à volume variable 22 contre l'embase 23.

La figure 6 montre d'une manière séparée tous ces éléments 22, 23 et 80 non montés. La figure 7 montre le récipient 21 monté, placé sur la base 29 du régulateur.

Pour assurer une bonne fixation de ces éléments on a prévu un bourrelet de bague 85 disposé sur le pourtour intérieur de celle-ci. En a sur la figure 6 on a montré plus en détail ce bourrelet. Ce bourrelet 85 coopère avec un bourrelet 87 d'embase situé à la partie supérieure et extérieure de l'embase 23 . Ceci est montré d'une manière agrandie en b à la figure 6. De cette manière lors du montage de la chambre la partie inférieure de celle-ci est coincée d'une manière étanche par ces deux bourrelets 85 et 87. On a prévu aussi un rebord 90 proéminent sur le pourtour extérieur de l'embase 23 constituant un taquet pour la bague 80 et le bord inférieur de la chambre 22.

L'invention n'est bien entendu nullement limitée à l'exemple décrit, donné à titre purement indicatif et que de nombreuses modifications peuvent être facilement apportées par l'homme de l'art sans pour autant sortir du cadre de l'invention.

## Revendications

1. Régulateur de pression (10) pour sonde d'intubation, canule de trachéotomie (1) ou instrument similaire destiné à la ventilation d'un patient, ledit instrument comportant un tuyau principal (2) ayant une première extrémité (2a) destinée à être insérée dans la trachée artère d'un patient (3) et une seconde extrémité (2b) destinée à être raccordée à un appareil respirateur (4), un ballonnet (5) annulaire gonflable d'étanchéité, qui entoure la première extrémité (2a) du tuyau principal (2), et au moins un tuyau auxiliaire (6) ayant une première extrémité raccordée au ballonnet d'étanchéité (5) et une seconde extrémité munie d'une valve (7) pouvant être raccordée à une source d'air comprimé (9) pour le gonflage du ballonnet d'étanchéité (5), ledit régulateur de pression comprenant un récipient (21) définissant une chambre à volume variable (22) ayant au moins un premier orifice (25) pour être raccordé audit tuyau auxiliaire (6) de telle façon que, en service, la chambre à volume variable (22) du régulateur de pression et le ballonnet d'étanchéité (5) soient en communication, ainsi qu'un moyen de pression agissant sur le récipient (21) pour exercer une certaine force au moyen d'une masse (50) pouvant se déplacer le long d'une tringle (38) articulée pour régler la pression de l'air contenu dans ladite chambre à volume variable, et par suite la pression de l'air contenu dans le ballonnet d'étanchéité (5) **caractérisé en ce qu'**il est prévu un système de verrouillage de ladite masse pour éviter son déplacement inopiné, système de verrouillage constitué par un élément vissable (60) muni d'une tête de friction (62) pour être mis en contact de blocage sur une partie de ladite tringle par un mouvement de rotation et d'une partie filetée (64) pénétrant dans le corps de la masse (50).

2. Régulateur selon la revendication 1 **caractérisé en ce qu'**un détecteur de déplacement (70) est rattaché à la tringle (38) pour fournir une indication de pression à un organe de gestion destiné à fournir sur un écran (74) l'indication de la pression et un historique des différentes opérations précédentes.

3. Régulateur selon l'une des revendications 1 ou 2 **caractérisé en ce que** le ou les tuyaux auxiliaires (6) comporte des moyens de détrompages basés notamment sur la couleur pour éviter les erreurs de branchement.

4. Régulateur selon l'une des revendications 1 ou 2 **caractérisé en ce que** les raccords des tuyaux auxiliaires (6) sont collés.

5. Régulateur selon la revendication 2 **caractérisé en ce que** l'organe de gestion (70) comporte des organes de mémoire pour contenir l'historique de son utilisation.

6. Système de respiration comportant un régulateur selon l'une des revendications 1 à 5 et un appareil respirateur (4).

7. Régulateur selon l'une des revendications 1 à 5 **caractérisé en ce que** le récipient (21) est formé d'une chambre à volume variable (22) et d'une embase (23), la chambre à volume variable (22) étant réalisée en une matière plastique souple et venant se glisser sur le bord supérieur de l'embase (23) reposant sur la base (29) du régulateur.

8. Régulateur selon la revendication 7 **caractérisé en ce qu'**il est prévu une bague (80) qui enserre la chambre à volume variable contre l'embase (23).

9. Régulateur selon la revendication 8 **caractérisé en ce qu'**il est prévu des bourrelets (85, 87) placés sur les pourtours de l'embase (23) et de la bague (80) en vue de coincer le bord inférieur de la chambre (22).

## Patentansprüche

1. Druckregulator (10) für Trachealtubus, Tracheotomiekanüle (1) oder ähnliches Instrument, das für die Beatmung eines Patienten bestimmt ist, wobei das Instrument einen Hauptschlauch (2) mit einem ersten Ende (2a) aufweist, das zum Einsetzen in die Luftröhre eines Patienten (3) bestimmt ist, und ein zweites Ende (2b), das zum Verbinden mit einem Beatmungsgerät (4) bestimmt ist, einen ringförmigen aufblasbaren Dichtungs-Cuff (5), der das erste Ende (2a) des Hauptschlauchs (2) umgibt, und mindestens einen Hilfsschlauch (6) mit einem ersten Ende, das mit dem Dichtungs-Cuff (5) verbunden ist, und einem zweiten Ende, das mit einem Ventil (7) ausgestattet ist, die mit einer Druckluftquelle (9) zum Aufblasen des Dichtungs-Cuffs (5) verbindbar ist, wobei der Druckregulator ein Behältnis (21) umfasst, das eine Kammer mit variablem Volumen (22) definiert, die mindestens eine erste Öffnung (25) für die Verbindung mit dem Hilfsschlauch (6) hat, so dass im Betrieb die Kammer mit variablem Volumen (22) des Druckregulators und der Dichtungs-Cuff (5) in Kommunikation sind, sowie ein Druckmittel, das auf das Behältnis (21) wirkt, um eine bestimmte Kraft mittels einer Masse (50) auszuüben, die sich entlang einer angelenkten Stange (38) verlagern kann, um den Druck der Luft, die in der Kammer mit variablem Volumen enthalten ist, und in der Folge den Druck der im Dichtungs-Cuff (5) enthaltenen Luft zu regeln, **dadurch gekennzeichnet, dass** ein Verriegelungssystem der Masse vorgesehen ist, um ihre unerwartete Verlagerung zu verhindern, wobei das Verriegelungssystem von einem schraubbaren Element (60) gebildet ist, das mit einem Reibungskopf (62) ausgestattet ist, um im Blockierkontakt auf einen Teil der Stange durch eine Rotationsbewegung gesetzt zu sein, und einem gewindeten Teil (64), der in den Körper der Masse (50) eindringt.

2. Regulator nach Anspruch 1, **dadurch gekennzeichnet, dass** ein Verlagerungsdetektor (70) der Stange (38) zugeordnet ist, um einem Verwaltungsorgan eine Druckangabe bereitzustellen, das bestimmt ist, auf einem Bildschirm (74) die Druckangabe und einen Verlauf der verschiedenen vorangehenden Operationen bereitzustellen.

3. Regulator nach einem der Ansprüche 1 oder 2, **dadurch gekennzeichnet, dass** der oder die Hilfsschläuche (6) Unverwechselbarkeitsmittel aufweisen, die insbesondere auf der Farbe basieren, um Anschlussfehler zu vermeiden.

4. Regulator nach einem der Ansprüche 1 oder 2, **dadurch gekennzeichnet, dass** die Anschlüsse der Hilfsschläuche (6) geklebt sind.

5. Regulator nach Anspruch 2, **dadurch gekennzeichnet, dass** das Verwaltungsorgan (70) Speicherorgane aufweist, um den Verlauf seiner Benutzung zu enthalten.

6. Atemsystem, aufweisend einen Regulator nach einem der Ansprüche 1 bis 5 und ein Atemgerät (4).

7. Regulator nach einem der Ansprüche 1 bis 5, **dadurch gekennzeichnet, dass** das Behältnis (21) von einer Kammer mit variablem Volumen (22) und einem Sockel (23) gebildet ist, wobei die Kammer mit variablem Volumen (22) aus einem elastischen Kunststoffmaterial hergestellt ist und über den oberen Rand des Sockels (23) gleitet, der auf der Basis (29) des Regulators ruht.

8. Regulator nach Anspruch 7, **dadurch gekennzeichnet, dass** ein Ring (80) vorgesehen ist, der die Kammer mit variablem Volumen gegen den Sockel (23) spannt.

9. Regulator nach Anspruch 8, **dadurch gekennzeichnet, dass** Wülste (85, 87) vorgesehen sind, die auf dem Umfang des Sockels (23) und des Rings (80) platziert sind, um den unteren Rand der Kammer (22) festzuklemmen.

## Claims

1. A pressure regulator (10) for an intubation probe, tracheostomy cannula (1) or similar instrument intended for ventilating a patient, said instrument including a main pipe (2) having a first end (2a) intended to be inserted into the trachea of a patient (3) and a second end (2b) intended to be coupled to a breathing apparatus (4), an inflatable annular sealing balloon (5), which surrounds the first end (2a) of the main pipe (2), and at least one auxiliary pipe (6) having a first end coupled to the sealing balloon (5) and a second end provided with a valve (7) which can be coupled to a compressed air source (9) for inflating the sealing balloon (5), said pressure regulator comprising a container (21) defining a variable volume chamber (22) having at least one first orifice (25) to be coupled to said auxiliary pipe (6) so that, in use, the variable volume chamber (22) of the pressure regulator and the sealing balloon (5) are in communication, as well as a pressure means acting on the container (21) to exert a certain force by means of a mass (50) which can be displaced along an hinged rod (38) to adjust the pressure of the air contained in said variable volume chamber, and subsequently the pressure of the air contained in the sealing balloon (5) **characterised in that** there is provided a system for locking said mass to prevent the inadvertent displacement thereof, a locking system consisting of a screwable element (60) provided with a friction head (62) to be in locking contact on a portion of said rod by a rotational movement and a threaded portion (64) entering the body of the mass (50).

2. The regulator according to claim 1, **characterised in that** a displacement detector (70) is attached to the rod (38) to provide a pressure indication to a management member intended to provide, on a screen (74), the indication of the pressure and a history of the different previous operations.

3. The regulator according to one of claims 1 or 2, **characterised in that** the auxiliary pipe(s) (6) includes/include foolproofing means based in particular on the colour in order to avoid the connection errors.

4. The regulator according to one of claims 1 or 2, **characterised in that** the couplings of the auxiliary pipes (6) are glued.

5. The regulator according to claim 2, **characterised in that** the management member (70) includes memory members to contain the history of the use thereof.

6. A breathing system including a regulator according to one of claims 1 to 5 and a breathing apparatus (4).

7. The regulator according to one of claims 1 to 5, **characterised in that** the container (21) is formed of a variable volume chamber (22) and a base plate (23), the variable volume chamber (22) being made of a flexible plastic material and sliding over the upper edge of the base plate (23) resting on the base (29) of the regulator.

8. The regulator according to claim 7, **characterised in that** there is provides a ring (80) which encloses the variable volume chamber against the base plate (23).

9. The regulator according to claim 8, **characterised in that** there are provided beads (85, 87) placed on the peripheries of the base plate (23) and the ring (80) in order to clamp the lower edge of the chamber (22).
